# EUROPEAN PATENT APPLICATION

(11) **EP 1 003 121 A2**
(43) Date of publication of application: **24.05.2000**
(21) Application number: 99121546.8
(22) Date of filing: 29.10.1999
(51) Int. Cl.: G06F 19/00

(54) **Medication and specimen management system**

(30) Priority: 30.10.1998 US 106411 P
(71) Applicant: Becton, Dickinson and Company, Franklin Lakes, New Jersey 07417 (US)
(72) Inventor: Savitz, Steven R., Teaneck, New Jersey 07666 (US); Moore, Neil, Hamilton, Ohio 45011 (US); Soares, Michael, Ridgewood, New Jersey 07450 (US); Cataldo, Renato, St. Louis, Missouri 63131 (US)
(74) Representative: von Kreisler, Alek, Dipl.-Chem.

(57) **Abstract**

A system and method to manage information relating to patients in a hospital environment. The system includes a plurality of information processing systems for processing information relating to a patient in the hospital, with each of the information processing systems being associated with a discrete hospital function. One or more portable data terminals for receipt and transmission of information relating to the patient are further provided. A control server is provided to communicate with each of the information processing systems and the portable data terminals, for transfer of information relating to the patient between the information processing systems and the portable data terminal. As such, the portable data terminal is capable of periodic communication with the control server for intermittent interfacing with the information processing systems for receiving information relating to the patient from the information processing systems through the control server, and for transferring data relating to the patient from the portable data terminal to the information processing systems through the control server.

## Description

### BACKGROUND OF INVENTION

### 1. Field of Invention

The present invention is relates to management of medication and specimens and procedures and patient information in a hospital environment. More particularly, the present invention relates to an improved system for integrating and managing information and data with respect to specimen collection, medication delivery and administration procedures.

### 2. Description of Related Art

In portable systems for identifying articles and printing identification relating to specific articles, an article is encoded by providing the article with a label having a bar code or similar identification thereon. A scanner, such as a laser scanner or an optical scanner, is capable of reading the encoded bar code information from the article, and processing the information.

Such portable systems are particularly useful in hospital environments, with respect to medication distribution and specimen sample collection procedures. For example, in a hospital environment, a phlebotomist is provided with a list of patients requiring blood collection for testing. The phlebotomist travels to the bedside of each patient requiring blood collection, draws a blood sample, and appropriately labels the sample with the patient identification and testing to be conducted.

Encoded identification of patients and testing procedures has simplified such collection procedures, and ensures proper correlation of the collected sample with the appropriate patient for analysis in a laboratory.

While portable data collection systems and devices are convenient for specimen collection management, it may be more efficient to integrate a plurality of hospital information systems such as laboratory systems, pharmacy systems, hospital administration systems, and the like. Such information and data is often stored in various hospital information systems, and communication between such systems can be difficult.

Accordingly,a need exists for a system which is capable of integrating and managing a plurality of hospital information systems, and transmitting data and information between such hospital information systems and portable devices for use at the patient's bedside.

### SUMMARY OF THE INVENTION

The present invention is a system for managing and administrating information. Desirably, the present invention is a system for management and administration of patient information in a hospital environment where information may be correlated between a plurality of information processing systems in a hospital environment.

Preferably, the system of the present invention includes a plurality of information processing systems for processing information relating to a patient in the hospital, with each of the information processing systems being associated with a discrete hospital function. The information processing systems may be selected from the group consisting of hospital information systems, laboratory information systems, pharmacy information systems, radiology information systems and accounting information systems. One or more portable data terminals for receipt and transmission of information relating to the patient are further provided. Also, a control server is in one-way or two-way communication with each of the information processing systems as well as the portable data terminals, for transfer of information relating to the patient between the information processing systems and the portable data terminal. The portable data terminal is capable of periodic communication with the control server for intermittent interfacing with the information processing systems for receiving information relating to the patient from the information processing systems through the control server, and for transferring data relating to the patient from the portable data terminal to the information processing systems through the control server. Information such as sampling data, medication administration and the like is communicated. Such communication is preferably accomplished by radio frequency transmission, ethernet, or serial connection.

The system may further include a terminal server or docking station associated with the portable data terminals, with the terminal server or docking station capable of providing two-way communication between the portable data terminals and the control server. Moreover, a local area network may be incorporated for communicating between the plurality of portable data terminals and the control server.

Preferably, the portable data terminal includes a microprocessor,reading means such as a bar code scanner, and printing means, with the reading means capable of reading identification information from a patient identification code and printing a corresponding information label, and the microprocessor capable of storing data relating to the identification information and the printed information label for transmission to the information processing systems through the control server.

The system of the present invention may further include means for gathering and managing information relating to the patient in the hospital comprising a plurality of information processing systems for processing information relating to the patient. The system includes a portable data terminal for collecting data relating to assessment of the patient, for example gathering of vital signs and the like. A control server in two-way communication with the information processing systems and the portable data terminal is further provided for transferring the data gathered from the patient by the portable data terminal to the information processing systems.

The system of the present invention further includes a method for managing patient information relating to a patient in the hospital. The method includes providing a plurality of information systems for processing information relating to patient and one or more portable data terminals. Information from the information systems is transferred to a control server, and accessed by the portable data terminals through the control server. Procedures are then conducted in accordance with the information accessed by the portable data terminals, and data relating to the procedures conducted is stored in the portable data terminals. Such stored data is then transferred from the portable data terminals to the information processing systems through the control server. Moreover, the transferring of information and data may be accomplished through a terminal server or docking system connected between the control server and the portable data terminal.

### DESCRIPTION OF THE DRAWINGS

FIG. 1 illustrates a block diagram of the patient information management system of the present invention.
FIG. 2 is a perspective view of a portable data terminal of the present invention.
FIG. 3 illustrates the patient information management system of the present invention.
FIG. 4 illustrates the patient information management system of the present invention.
FIG. 5 illustrates the patient information management system of the present invention.
FIG. 6 is a flow chart of the method to the present invention.

### DETAILED DESCRIPTION

The system of the present invention is described herein for use in a hospital environment. It is understood, however, that the system is useful for a large variety of environments, and is therefore not meant to be limited to a hospital environment. Furthermore, it is understood that each information system as discussed herein may be physically located within the laboratory within a hospital, or may be located outside of the hospital such as an outside laboratory contracted to conduct analyses for the hospital.

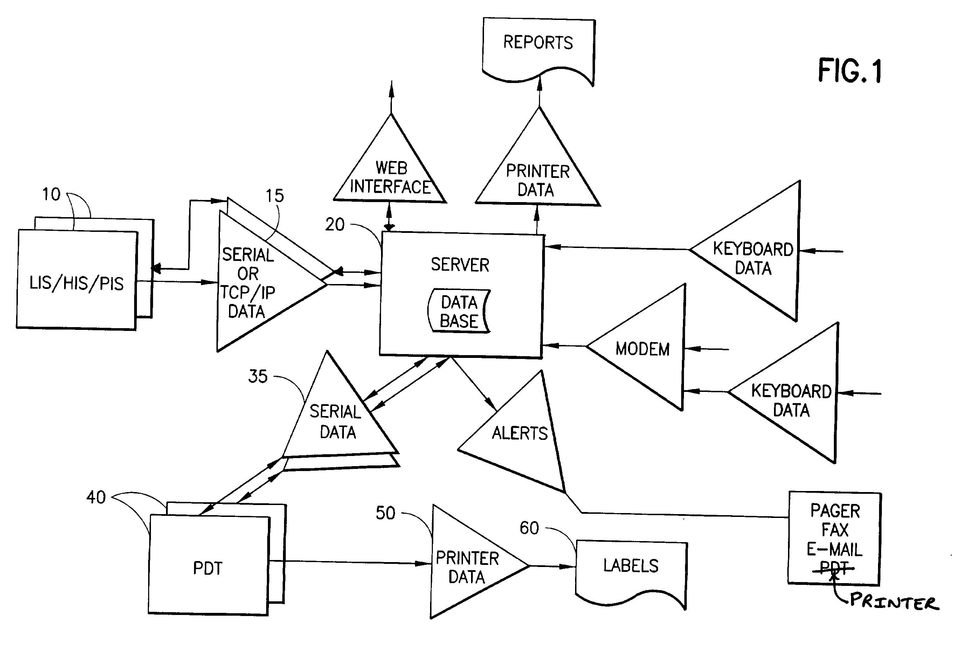

The system of the present invention is illustrated in the block diagram of FIG. 1. The system includes an information processing system, preferably a plurality of information processing systems **10**, for processing and managing information. The information processing systems may be, hospital administration systems, laboratory information systems, pharmacy information systems, accounting information systems, and the like. The hospital administration system may include an entire patient history file listed for a specific patient, including all medical records, for a particular visit and/or over the lifetime of the patient. The laboratory information system may include information relating to specific laboratory analyses which have been conducted on samples of a particular patient. The pharmacy information system may include information relating to medications which have been prescribed and/or administered for a particular patient. The accounting information system may include accounting information relating to all procedures and tests conducted on a particular patient for billing and insurance purposes.

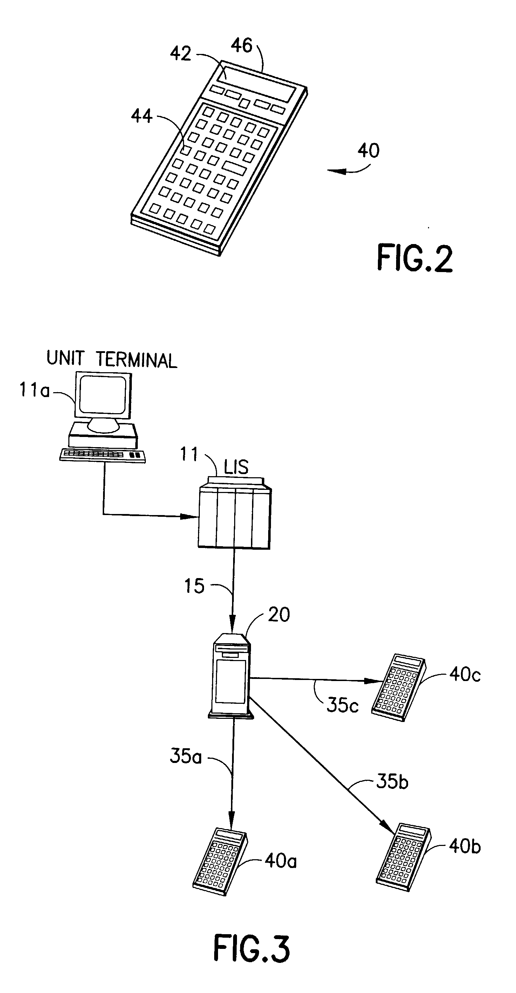

The system of the present invention further includes one or more portable data terminals **40** as shown in FIGS. 1 and 2. Portable data terminal **40** can be any type of portable device useful in gathering and transmitting information, such as information relating to patient well-being within a hospital environment. The portable data terminal includes a microprocessor having memory for storage of information and data. Portable data terminals **40** includes a keypad **44** for manual entry of data by a user, a display **42** for visual communication with a user. Portable data terminal **40** also includes a bar code scanner **46** for reading information, including patient identification information from a patient identification bracelet as well as container information from a manufacturing identification on a sample container label or medication container. Portable data terminal **40** may also include printing means, for printing a label for attachment to a sample container, including information such as patient identification information or laboratory analyses to be conducted on the sample.

A control server **20** in two-way communication with each of information processing systems **10**, and with each of the one or more portable data terminals **40**. Through such communication, the control server **10** can manage information relating to the patient by transferring such information between each of information processing systems **10**, between each of portable data terminals **40**, as well as between information processing systems **10** and portable data terminals **40.**

Two-way communication between control server **20** and information systems **10** can be any type of communication link for transfer of information and data. As shown in FIG. 1, an information processing system serial connection **15**, serially links information processing systems **10** with control server **20** for transmission of data. The two-way communication between control server **20** and portable data terminals **40**, as terminal serial connection **35**, can be the same or different type of communication link for transfer of information and data. Such a connection can be an electrical connection, such as an ethernet connection, a telephone connection using a data-over-voice modem, a wireless connection such as a radio frequency transmission, and the like.

Information processing systems **10** may further include one or more peripheral devices in communication therewith. Information processing systems **10** may include unit terminals for input of data, printers for generation and printing of reports, communication links such as pagers, printers, facsimile machines, and other similar peripheral elements which can be used in administration and management of information within the system. Control server may also include peripheral devices and elements for administration and management of information within the system.

Interconnection of the components of the system of the present invention is shown in FIGS. 3, 4 and 5. FIG. 3 shows an information processing system **10** that includes a laboratory information system **11** (LIS) in two-way communication with control server **20**. Information and data is entered into laboratory information system **11** by a laboratory terminal **11a**. Such information and data is then processed and stored in laboratory information system **11**.

Multiple portable data terminals **40**, further depicted as **40a**, **40b** and **40c**, are also included in the system, in two-way communication with control server **20**. Such multiple portable data terminals **40** are capable of interfacing and transferring information between information processing systems **10** and portable data terminals **40** through control server **20**. The two-way communication link is established between control server **20** and each of portable data terminals **40** using a docking station, in which portable data terminals **40** can be removably "docked" for linking with control server **20** for periodic interfacing therewith, thereby providing a direct two-way communication link for transfer of information and data therebetween.

Each of portable data terminals **40a**, **40b** and **40c** are capable of transport within a hospital environment, for example to a patient's bedside for assessment of patient medical characteristics and conditions. For example, portable data terminals **40a**, **40b** and **40c** may be capable of gathering and storing data relating to procedures conducted, such as gathering of vital signs of a patient including body temperature, blood pressure, and the like, confirmation of sampling of patient specimens, or may be capable scanning identification information of a patient from a bar code bracelet for printing of an identification label for a specimen container label or verifying the correct administration of a medication.

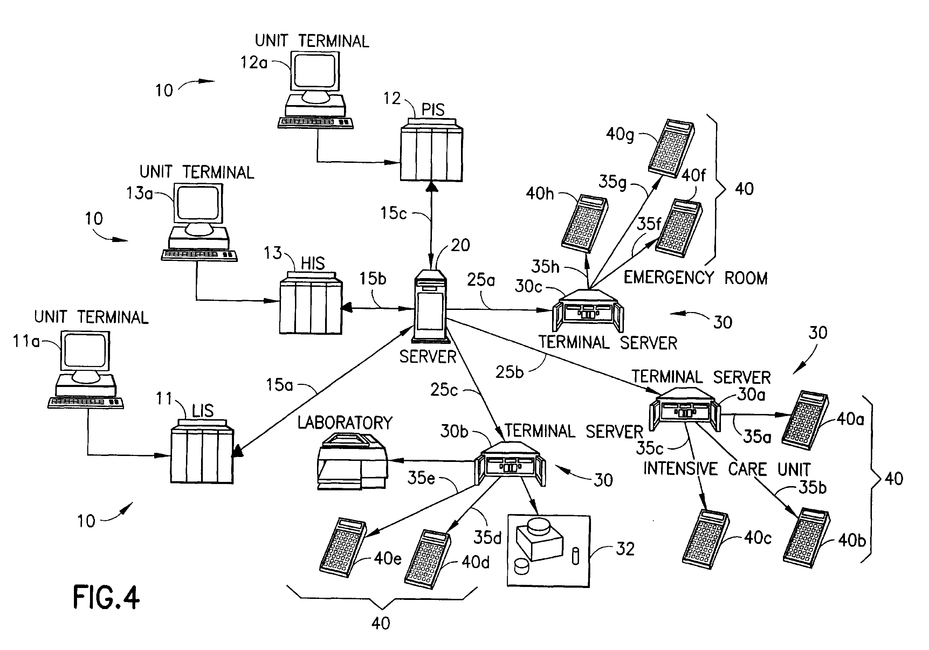

In a further embodiment as depicted in Figure 4, a plurality of information processing systems **10** may be included in the system, including laboratory information system **11** (LIS), pharmacy information system **12** (PIS) and hospital administration system **13** (HIS). Each of these information processing systems **11**, **12** and **13** may include one or more unit terminals such as laboratory terminal **11a**, pharmacy terminal **12a**, and hospital terminal **13a**, such as a user station with a keyboard for data entry of relevant system information by a user. Each of these plurality of information systems is in two-way communication with control server **20** through information serial connections **15a**, **15b** and **15c**, respectfully. Such information serial connections **15a**, **15b** and **15c** provide direct, two-way communication of information and data between laboratory information system **11**, pharmacy information system **12**, and hospital administration system **13**. Moreover, each of laboratory information system **11**, pharmacy information system **12**, and hospital administration system **13** are capable of intercommunicating with each other through the communication link maintained through control server **20**.

Portable data terminals **40**, further depicted as **40a**, **40b**, **40c**, **40d**, **40e**, **40f**, **40g** and **40h**, are provided as described above for gathering of data and information relating to patients within a hospital environment. These portable data terminals are in two-way communication with control server **20**. Such two-way communication may be established by a direct communication. Preferably, such two-way communication extends through terminal servers **30** serving a specific hospital facility, for example, terminal server **30a** for an intensive care unit, terminal server **30b** for a hospital laboratory, and terminal server **30c** for an emergency room. In such an embodiment, control server **20** is linked to terminal server **30a**, **30b**, and/or **30c** through terminal serial connection **25a**, **25b**, **and 25c**, respectively. Portable data terminals **40a**, **40b**, **40c**, **40d**, **40e**, **40f**, **40g** and **40h** are in two-way communication with the respective terminal server **30a**, **30b** and **30c**, thereby providing two-way communication between control server **20** and each of portable data terminals **40a**, **40b**, **40c**, **40d**, **40e**, **40f**, **40g** and **40h** through terminal servers **30a**, **30b** and **30c**. Preferably, such two-way communication between control server **20** and each of portable data terminals **40a**, **40b**, **40c**, **40d**, **40e**, **40f**, **40g** and **40h** is established through a docking station as discussed above, with the docking station located at terminal servers **30a**, **30b** and **30c**.

Moreover, terminal servers **30a**, **30b** and **30c** are preferably located at various locations within the hospital environment to provide ease of access. For example, terminal server **30a** which serves an intensive care unit in a hospital may be located at a central nursing station for that intensive care unit. Such an arrangement is particularly useful when terminal server **30a** includes a docking station for interfacing with portable data terminals **40a**, **40b** and **40c**, in that when orders are received by portable data terminals **40a**, **40b** and **40c** through terminal server **30a**, as will be discussed in more detail herein, the nursing station can be immediately notified of such orders at a centralized location.

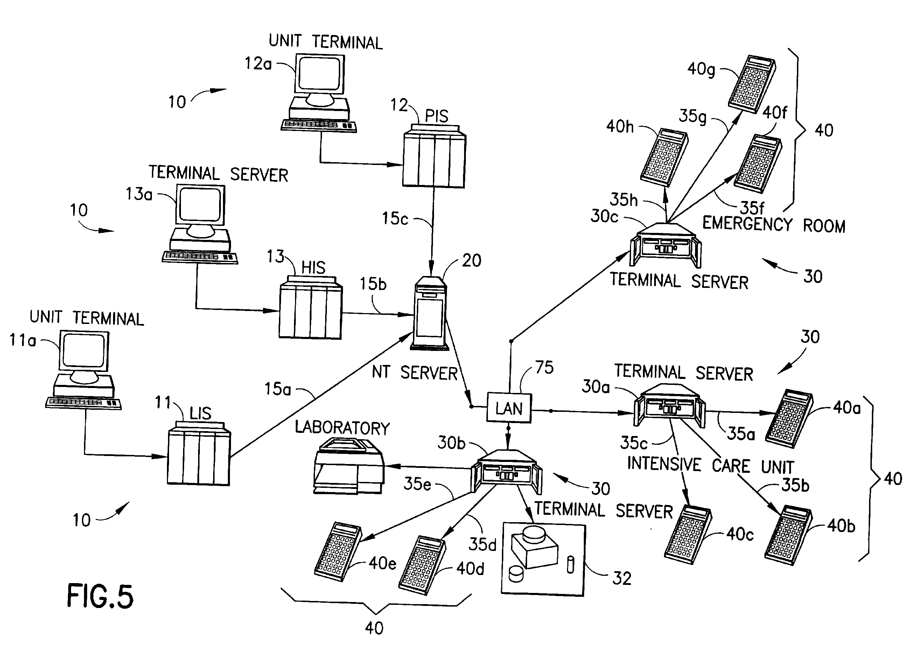

In an alternate embodiment shown in FIG. 5, the two-way communication between control server **20** and each of terminal servers **30a**, **30b**, and **30c** may be accomplished through a local area network **75**, capable of interconnecting and networking a plurality of terminal servers for communication there between and independently with control server **20**.

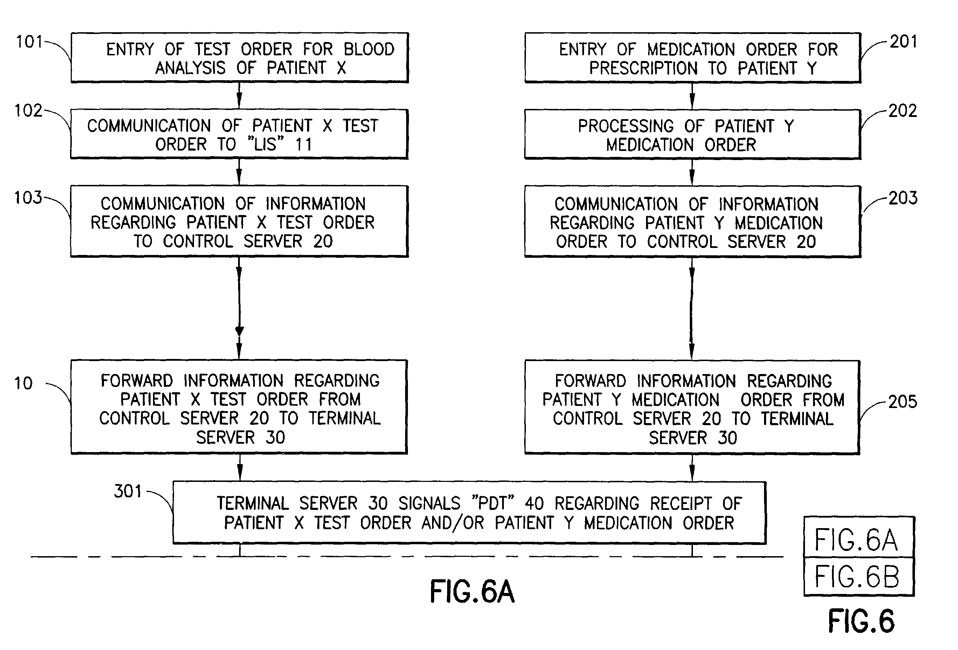

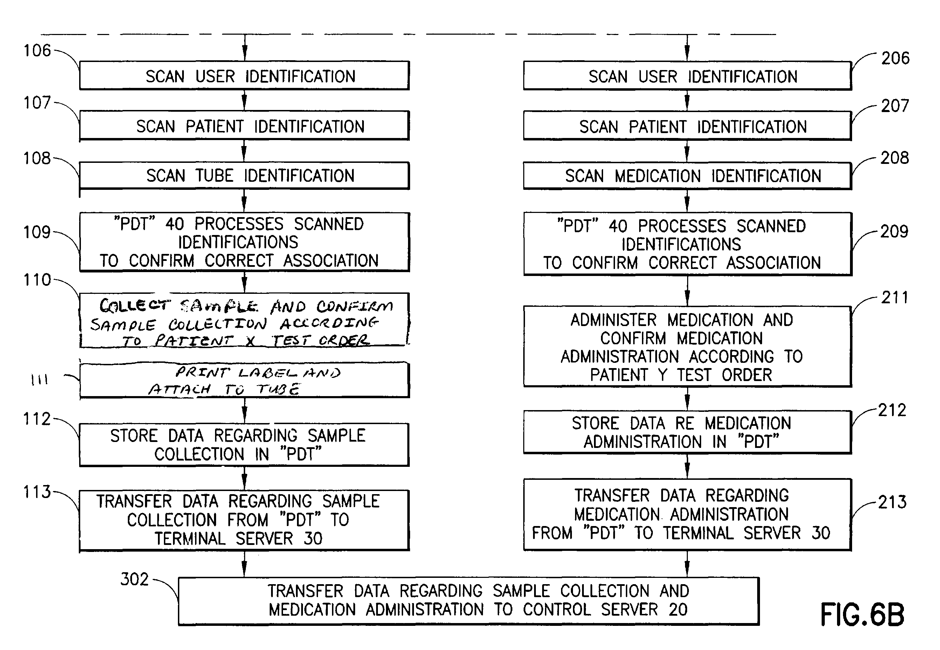

Referring to FIG. 4, the system will now be described in terms of a preferred method of use, with additional reference to the flow chart of FIG. 6. In a typical hospital environment, a patient history file is maintained for each patient within the hospital environment in a hospital information system, such as hospital information system **13**. Such a patient history file may include an entire medical history of a patient, or may include only current information relating to a current patient hospital admission. Such patient history file is typically input into the hospital information system **13** by user data entry at hospital terminal **13a**. For example, upon admission to the hospital, a clerk may input various medical information into the hospital information system **13**, such as patient identification information, allergies, medical history, etc. At the time of patient admission, the patient is typically provided with a patient identification, such as a bar code identification bracelet to be worn by the patient, which includes patient identification information encoded thereon. Such patient identification information is further stored in the patient history record of hospital information system **13**.

Typically, during a patient's hospital stay, it is necessary to obtain biological samples from a patient in order to conduct various analytical tests. In the present system, when such a test is ordered, a test order is entered into laboratory information system **11**. Such test order may be entered directly into laboratory information system **11** through laboratory terminal **11a**, or may be entered into hospital information system **13** through hospital terminal **13a**, and communicated to laboratory information system **11** through control server **20**. For example, as shown at step **101** of FIG. 6, a doctor may order a test order for a blood analysis of Patient X, which test order is entered into hospital information system **13** to maintain a complete record of all tests performed on Patient X. Further, the test order is communicated to laboratory information system **11** for completion of the test order for a blood analysis of Patient X, as in step **102**.

Information relating to the test order for a particular patient or patients is then communicated to control server **20** at step **103**, for forwarding to the appropriate portable data terminal **40**. For example, laboratory information system **11** processes the test order for a blood analysis of Patient X, and determines that a blood sample must be obtained from Patient X in a specific blood collection container in order to conduct the appropriate blood analysis according to the test order. Laboratory information system **11** communicates information relating to the blood sample to be obtained from Patient X to control server **20**, for obtaining the appropriate blood sample in the appropriate container.

Preferably, as discussed above, control server **20** communicates with portable data terminals **40a**, **40b**, **40c**, **40d**, **40e**, **40f**, **40g** and **40h** through terminal servers **30a**, **30b**, and **30c**, which can be located at specific hospital facilities, for example at a nursing station for the intensive care unit. In this manner, communication between control server **20** and portable data terminals **40a**, **40b** and **40c**, is accomplished through terminal server **30a**, for example by using terminal server **30a** as a docking station for portable data terminals **40a**, **40b** and **40c**. Control server **20** may forward the information relating to the test order directly to all of the portable data terminals **40a**, **40b**, **40c**, **40d**, **40e**, **40f**, **40g** and **40h** within the hospital environment, preferably through the terminal servers **30a**, **30b** and **30c**. Alternatively, control server **20** may access information from hospital information system **13** regarding the location of the patient or patients to which the test order relates, and forward the information relating to the test order only to that specific portable data terminal or terminal server at the facility location where that patient or patients are located. For example, hospital information system **13** may include information identifying the location of each patient within a hospital. When the test order for a blood analysis of Patient X is communicated to control server **20** from laboratory information system **11**, control server **20** may access hospital information system **13** at step **104** to determine the location of Patient X. The hospital information system **13** can then communicate to control server **20** that Patient X is in the intensive care unit of the hospital. Control server **20** can then communicate with terminal server **30a** in the intensive care unit, for transfer of information relating to the test order for Patient X at step **105**.

In a similar manner, a particular patient may be prescribed certain medication during hospital visit. In the present system, information relating to the prescription and administration of such medication is entered into pharmacy information system **12** in the form of a medication order, either directly through pharmacy terminal **12a**, or through hospital terminal **13a**. For example, a doctor may prescribe a dosage of antibiotics to be administered to Patient Y. Such prescription is entered into the pharmacy information system **12** in the form of a medication order at step **201**, and pharmacy information system can process the medication order at step **202**, for example to determine whether Patient Y has any allergies to the specific antibiotic in the medication order. Alternatively, pharmacy information system **12** may communicate the medication order to hospital information system **13**. With hospital information system **13** maintaining a patient history file for Patient Y, hospital information system **13** can process the medication order, for example to determine whether Patient Y has any allergies to the specific antibiotic in the medication order. Should the medication order be acceptable for Patient Y, hospital information system **13** can inform pharmacy information system **12** appropriately.

Information relating to the medication order for a particular patient or patients is then communicated to control server **20** at step **203**, for forwarding to all of the portable data terminals **40a**, **40b**, **40c**, **40d**, **40e**, **40f**, **40g** and **40h**, or to the appropriate portable data terminal **40** preferably through terminal server **30a** as in step **205**, in a similar manner as discussed with reference to the test order.

When control server forwards information relating to the test order and/or medication order to terminal server **30a**, terminal server **30a** signals one or all of portable data terminals **40a**, **40b** and **40c** docked within terminal server **30a**. Such signal can be, for example, in the form of an audio signal such as an alarm, a visual signal such as a blinking light or a readout on display **42** of the portable data terminal, or a combination of both an audio and visual signal. The user, such as a nurse in the intensive care unit, detects the signal received by the portable data terminal **40a**, **40b** and/or **40c**. The nurse then retrieves one of the portable data terminals, such as portable data terminal **40a**, from the docking station of terminal server **30a** for completion of the test order and/or medication order communicated to portable data terminal **40a**. For example, control sewer **20** may forward the test order for a blood sample of Patient X and the medication order for administration of an antibiotic to Patient Y, to terminal server **30a**, as both Patient X and Patient Y are located in the intensive care unit. As in step **301**, terminal server **30a** signals portable data terminal **40a** that an order has been received. Nurse Z retrieves portable data terminal **40a** from terminal server **30a**. Display **42** of portable data terminal **40a** indicates that a blood sample is required from Patient X and that an antibiotic is to be administered to Patient Y. Nurse Z then performs sampling rounds and medication rounds, either individually or at the same time, according to the orders communicated to the portable data terminal **40a**.

In order to perform sampling rounds, display **42** of portable data terminal **40a** may indicate to Nurse Z which patient requires sampling, for example Patient X. During sampling rounds, Nurse Z travels to Patient X to obtain a blood sample. At bedside, Nurse Z may first optionally scan a user identification code with scanner **46** of portable data terminal **40a** at step **106**. Such user identification code may be an employee identification tag bearing a bar code identification of Nurse Z. Scanner **46** of portable data terminal **40a** is then used to scan a patient identification code of Patient X at step **107**, such as an identification bracelet provided to Patient X during admission to the hospital. The microprocessor of portable data terminal **40a** processes the scanned patient identification, and confirms that a blood sample is to be obtained from Patient X. Nurse Z then selects the appropriate collection container for the sample to be taken, such as a vacuum blood collection tube having color-coded stopper, representing a specific test to be conducted on the sample. The collection tube may include a tube identification such as a manufacturing bar code identification, which identifies the type of collection tube. Scanner **46** of portable data terminal **40a** can then be used to scan the collection tube bar code identification at step **108**, with the microprocessor of portable data terminal **40a** processing the scanned information to confirm that the appropriate collection tube has been selected for the particular blood sample to be taken for Patient X, as in step **109**. Portable data terminal **40a** may then provide confirmation to Nurse Z that the correct collection tube has been selected, and that the blood sample can be collected. Nurse Z then obtains the blood sample and communicates to portable data terminal **40a** that the blood sample has been collected according to the test order, as in step **111**. Such communication can be in any manner, preferably by data entry by Nurse Z through keypad **44**. Preferably, portable data terminal **40a** includes printer **50**, which prints a collection tube label at step **111** for attaching to the sample collection tube. The information printed on the collection tube label may include any appropriate sampling information, for example, patient identification, sampling date and time, user identification, tests conducted, as well as any other appropriate information, and is preferably printed in both alphanumeric form and bar code form. Such information is also preferably stored as sampling data in the memory of the microprocessor of portable data terminal **40a**.

The collection tube including Patient X's blood sample is then forwarded to the laboratory for completion of appropriate analyses according to the test order. Portable data terminal **40a** can then communicate the next test order to Nurse Z, or can indicate that all test orders have been completed.

In order to perform medication rounds, display **42** of portable data terminal **40a** may indicate to Nurse Z which patient requires administration of medication, for example indicating that Patient Y requiring administration of antibiotics. During medication rounds, Nurse Z travels to Patient Y to administer antibiotics. At bedside, Nurse Z may again first optionally scan an employee identification tag bearing a bar code identification of Nurse Z at step **206**. Scanner **46** of portable data terminal **40a** then scans Patient Y's bracelet identification code at step **207**. The microprocessor of portable data terminal **40a** processes the scanned patient identification, and confirms that antibiotics are to be administered to Patient Y. Nurse Z then selects the appropriate antibiotics, typically provided by the pharmacy. The medication may include an identification, preferably a bar code label, which identifies the medication and may include the identification of the patient to whom the medication is to be administered. Scanner **46** of portable data terminal **40a** can then be used to scan the bar code label of the medication administration container **208**, with the microprocessor of portable data terminal **40a** processing the scanned information to confirm that the medication is to be administered to Patient Y at step **209**. Portable data terminal **40a** may then provide confirmation to Nurse Z that the medication and the patient are appropriate, and Nurse Z may then administer the medication to Patient Y. After administering the medication, Nurse Z may communicate to portable data terminal **40a** that the medication has been administered according to the medication order, as in step **211**. Data relating to administration of the medication and completion of the medication order is then stored in the memory of the microprocessor of portable data terminal **40a**.

Once all test orders and medication administrations (as well as any other procedures communicated through portable data terminal **40a**) have been completed, portable data terminal **40a** is returned to the docking station **30a** for interfacing with control server **20** therewith. The data stored in the memory of portable data terminal **40a** is then downloaded or transferred to the appropriate information system through control server **20** as in step **312**. For example, sampling data relating to the blood sample collected from Patient X is transferred to control server **20**, and may be communicated to laboratory information system **11**. Likewise, medication data relating to administration of the antibiotic to Patient Y is transferred to control server **20**, and may be communicated to pharmacy information system **12**. Such data may also be communicated to other information processing systems **10,** for example an accounting information system for billing purposes, a hospital information system **13** for updating of the patient history file, an inventory control system for controlling inventory of medication, equipment, and the like.

As indicated above, the blood sample collected from Patient X according to the test order is forwarded to the laboratory for analysis. Such a laboratory may be on-site within the hospital facility, or may be an outside testing laboratory. The laboratory may also include portable data terminals **40d** and **40e** in communication with control server **20**, for example, through terminal server **30b**. When the laboratory receives the blood sample, scanner **46** of one of portable data terminals **40d** and **40e** may be used to scan the identification label of the sample collection tube, in order catalog receipt of the sample by the laboratory and transfer data relating to receipt of the blood sample to the laboratory information system **11**, through control server **20**. Analytical testing according to the test order can then be undertaken by a laboratory Technician Q.

During analysis of the sample, Technician Q may scan with scanner **46** of portable data terminal **40d** an employee identification code as done with Nurse Z above, in order to record data relating to the technician performing analysis of the sample. Testing of the blood sample according to the test order can then be undertaken, for example, employing various analytical instrumentation **32** within the laboratory. Preferably, such analytical instrumentation **32** is in communication with control server **20**, for example through terminal server **30b**. In this manner, data relating to the results of the analytical testing of the blood sample can be automatically recorded and transferred to the appropriate information processing system such as laboratory information system **11** by analytical instrumentation **32** through terminal server **30b**. Alternatively, data relating to the results may be input into portable data terminal **40d** by Technician Q, which data can then be transferred to the appropriate information processing system through terminal server **30b** and control server **20**.

## Claims

1. A system for managing information relating to a patient in a hospital environment comprising:
a plurality of information processing systems for processing said information relating to said patient, each of said plurality of information processing systems being associated with a hospital function;
a portable data terminal for receipt and transmission of said information relating to said patient; and
a control server in communication with each of said plurality of information processing systems and said portable data terminal for managing said information relating to said patient.

2. A system as in claim 1, wherein said plurality of information processing systems are selected from the group consisting of hospital information systems, laboratory information systems, pharmacy information systems, radiology information systems and accounting information systems.

3. A system as in claim 1, further comprising a plurality of portable data terminals, wherein said control server is in communication between each of said plurality of information processing systems and each of said plurality of data terminals for managing said information relating to said patient.

4. A system as in claim 1, further comprising a terminal server associated with said portable data terminal, said terminal server providing communication between said portable data terminal and said control server.

5. A system as in claim 4, further comprising a plurality of terminal servers each associated with a plurality of portable data terminals, said plurality of terminal servers each providing communication between said plurality of portable data terminals and said control server.

6. A system as in claim 5, wherein said communication between said plurality of portable data terminals and said control server is conducted through a local area network.

7. A system as in claim 1, wherein said portable data terminal includes a microprocessor, reading means, and printing means, said reading means capable of reading identification information from a patient identification code and printing a corresponding information label, and said microprocessor capable of storing data relating to said identification information and said printed information label for transmission to said plurality of said information systems through said control server.

8. A system as in claim 7, wherein said reading means is a bar code scanner.

9. A system as in claim 1, wherein said plurality of information processing systems are in communication with each other through said control server.

10. A system as in claim 1, wherein said portable data terminal is capable of periodic communication with said control server for intermittent interfacing with said plurality of information processing systems for receiving said information relating to said patient from said plurality of information processing systems through said control server and for transferring data relating to said patient from said portable data terminal to said plurality information processing systems through said control server.
